# EUROPEAN PATENT SPECIFICATION

(11) **EP 1 926 995 B1**
(45) Date of publication and mention of the grant of the patent: **04.04.2018**
(21) Application number: 06779456.0
(22) Date of filing: 15.09.2006
(51) Int. Cl.: G01N 33/569

(54) **A METHOD OF SCREENING MHC MOLECULES**
SCREENING-VERFAHREN FÜR MHC-MOLEKÜLE
METHODE DE CRIBLAGE DE MOLECULES DE CMH

(30) Priority: 19.09.2005 GB 0519029
(43) Date of publication of application: 04.06.2008
(73) Proprietor: Prolmmune Limited, Oxford, Oxfordshire OX4 4GA (GB)
(72) Inventor: SCHWABE, Nikolai, Franz, Gregor, Oxford OX2 6BP (GB); TAN, Linda, Cheng-Choo, Oxford OX2 6BP (GB); GOUVEIA, Catherine, Elizabeth, Witney OX28 3UD (GB)
(74) Representative: Vossius & Partner Patentanwälte Rechtsanwälte mbB
(86) International application number: PCT/GB2006/003444
(87) International publication number: WO 2007/034151

(56) References cited:
- WO-A-02/30964
- WO-A-99/21572
- WO-A-2005/047902
- US-A1- 2003 073 102
- US-A1- 2003 124 613
- US-A1- 2004 072 262
- ADMON ARIE ET AL: "Tumor antigens and proteomics from the point of view of the major histocompatibility complex peptides." MOLECULAR AND CELLULAR PROTEOMICS, vol. 2, no. 6, June 2003 (2003-06), pages 388-398, XP002435525 ISSN: 1535-9476
- KHILKO SERGEI N ET AL: "Direct detection of major histocompatibility complex class I binding to antigenic peptides using surface plasmon resonance: Peptide immobilization and characterization of binding specificity" JOURNAL OF BIOLOGICAL CHEMISTRY, vol. 268, no. 21, 1993, pages 15425-15434, XP002435526 ISSN: 0021-9258
- CARLSÉN S ET AL: "Cartilage oligomeric matrix protein (COMP)-induced arthritis in rats." CLINICAL AND EXPERIMENTAL IMMUNOLOGY DEC 1998, vol. 114, no. 3, December 1998 (1998-12), pages 477-484, XP002435527 ISSN: 0009-9104
- C. Sylvester-Hvid ET AL: "Establishment of a quantitative ELISA capable of determining peptide - MHC class I interaction", Tissue Antigens, vol. 59, no. 4, 1 April 2002 (2002-04-01), pages 251-258, XP55006262, ISSN: 0001-2815, DOI: 10.1034/j.1399-0039.2002.590402.x

## Description

The present invention relates to a method of screening MHC molecules, and more precisely to a method of screening MHC binding peptides, and kits for use in said method.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells. MHC molecules typically contain three "constituent peptides": an MHC alpha and MHC beta chain, and an MHC binding peptide bound in a groove formed by the alpha and beta chains when properly folded.

In the above interaction of the MHC molecule and the T cell, the T cell recognizes and binds to the peptide bound in the MHC binding groove and the surrounding area of the MHC binding groove itself. In order for a peptide to bind to an MHC molecule and therefore be able to cause an immune response *in vivo* it needs to fit the properties of the binding groove, which are also referred to as the "binding motif", of the relevant MHC allele in question, which restricts the type of peptides that can bind to a given MHC allele.

Understanding, which peptides in a protein sequence can bind to a given MHC molecule and cause an immune response, is of considerable interest for understanding cellular immunity and in order to design and monitor the effectiveness of immunotherapeutic products, such as vaccines.

Peptide binding to Class I or Class II MHC molecules is non-covalent. It is known that class I MHC molecules typically bind peptide fragments of 8 to 11 amino acid length in their binding groove formed between the MHC class I alpha1 and alpha2 domains. In rare cases MHC binding peptides with longer sequences, e.g. up to 13 mer sequences have also been reported.

For Class II MHC molecules binding of the peptides occurs in the groove formed between the alpha1 and beta1 domains of the molecule, the length of binding peptide varies more widely, and these peptides are typically 15 to 25 amino acids long or even longer, e.g. up to 30 amino acids length. In any event, however, typically only short fragments of full-length proteins will bind to Class I or Class II MHC Molecules.

Various processes have been developed for identifying new MHC binding peptides that may be T cell epitopes and many experimental methods start with constructing an overlapping library of peptide fragments from a given protein sequence, by synthesising a constant length (n-mer) amino acid sequences which are offset from one another along the protein sequence by fixed number of amino acids. The MHC binding properties and potential for activating T cells of each sequence can then be assessed in a number of assays.

The T2 binding assay (Elvin, J. et al. (1993), J. Immunol. Meth. 158:161-171) uses T2 cells that express a desired MHC allele. These MHC molecules will have bound peptides endogenous to the T2 cells on the cell surface, which can be "stripped" off these MHC molecules by incubating the T2 cells at low pH, e.g. pH 2-3 for a short period. The affinity of a putative MHC binding peptide for the presented MHC allele can be determined when the cells carrying the MHC molecules are incubated with a putative MHC binding peptide, beta 2 microglobulin and a labelled conformational antibody, and the binding of the putative MHC binding peptide is compared to the binding of a test peptide with a known affinity. Although this method has been known to produce good results for determining the binding affinity of peptides relatively accurately, it is very labour intensive, involves cell culture and is intrinsically not very scaleable.

The enzyme linked immunospot ELISpot assay such as described in EP0941478 can measure the activation of immobilized T cells in a sample by detecting cytokines secreted by T cells in response to stimulation with a relevant peptide antigen through antigen presenting cells through capture of the cytokines secreted by responding T cells in their vicinity on an adsorber membrane with a cytokine-specific monoclonal antibody. The cytokine capture is then detected with a second anti-cytokine antibody, which binds to a different epitope on the same cytokine compared to the capture antibody. Binding of the second antibody is then detected with a colour-based labelling reaction. As a consequence, stimulated cells are then detected as coloured spots on the membrane, which form around the original location where the cell was immobilized, The disadvantage of the ELISpot method is that it is fairly labour intensive and reading out the resulting plates accurately requires high skill from the operator or sophisticated software. More importantly, however the antigen presentation in this assay is usually carried out through regular antigen presenting cells, which by their nature will usually have six different class I and six different class II MHC alleles available for presentation. It is therefore difficult using this method to establish the accurate allelic MHC restriction for an antigen that is found to cause stimulation.

Existing MHC binding peptides that have been identified with the methods outlined above and other methods, such as crystallographic analysis of the conformation of and charge distribution in the MHC binding groove has led to binding motifs being defined for the most common MHC alleles, setting rules for what type of putative MHC binding peptide can actually bind well to MHC molecules of a given allele. These motifs have been translated into predictive computer algorithms for predicting peptide binding to MHC molecules such as the SYFPEITHI algorithm (Rammensee H.-G., et al. (1995), Immunogenetics 41:178-228). It is known that while these algorithms will successfully predict a number of high likelihood binding candidates, they also miss out a lot of binding peptides which do not match the classical binding motifs well, but which can still bind well. Further accurate binding prediction is not a conclusive test as to whether an MHC binding peptide is presented in vivo where it may not be loaded onto the relevant MHC-molecules for other physiological reasons.

The ultimate test for the relevance of any putative MHC binding peptide antigen is not just whether or not it can bind to an MHC molecule, but whether T cells with T cell receptors restricted to this peptide-MHC combination actually exist in the vertebrate species of interest, i.e. whether the peptide is a T cell epitope.

It has been established that isolated or recombinant forms of MHC-peptide molecules are useful for detecting, separating and manipulating T cells according to the specific peptide antigens these T cells recognize. It has also been understood that the interaction between MHC molecules and TCRs across cell surfaces is multimeric in nature and that the affinity of a single MHC molecule for a given TCR is generally quite low. As a consequence, there has been an effort to develop multimeric forms of isolated or recombinant MHC-peptide molecules that have an increased functional avidity in order to make such molecules more useful in the applications described above. Various methods are known from the art to purify protein complexes and especially MHC molecules.

As a consequence MHC multimers, such as MHC peptide tetramers-(see EP812331) or MHC peptides pentamers (see WO2004/018520) have been developed in order to label and detect, e.g. by flow cytometry, T cells in a sample that react to an MHC binding peptide of interest. While using these MHC multimers with the required MHC binding peptides in question can give the definitive proof as to whether a specific peptide is a T cell epitope, it would be very labour intensive and time consuming to attempt to construct fully purified functional MHC multimers for a large number of peptides, e.g. every candidate peptide in a protein sequence. As a consequence, MHC multimers have been used primarily in the past only to confirm MHC binding peptides as T cell epitopes, once these binding peptides had already been selected from a larger candidate list through another screening method such as the ones described above.

Recently, a high-throughput synthesis method for providing MHC multimers for a large number of binding peptides very quickly and cost effectively, has been developed; patent publication GB 2424645 B. While this significantly raises the utility of MHC oligomers in T cell epitope screening applications it would still be a lot of effort, even with this significantly accelerated and simplified method, to screen a very large number of peptides, such as 100s and 1000s of peptides, where their MHC binding characteristics are not known In this case it would be likely that the synthesis of the MHC oligomers will fail for most putative MHC binding peptides due to their insufficient binding affinity and therefore a significant proportion of the effort in the synthesis process would be wasted.

Commonly, the number of potential MHC binding peptides to be screened in a given project will usually be large (e.g. several hundreds of overlapping peptides in a typical length protein). Recent methods that have been developed to screen larger numbers of peptides are disclosed in WO 2004/034033, WO 2005/010026 or WO 2005/047902.

WO 2005/010026 provides methods for determining activation of a peptide-specific T cell by a MHC-binding peptide bound to an MHC monomer by incubating under suitable conditions peptide-specific T cells on a solid surface having attached thereto a plurality of MHC monomers having an MHC-bindiug peptide for which the T cells are specific; and an antibody specific for a selected surface T cell activation marker. The incubation allows formation of an antibody-surface activation marker complex on the surface of at least one of the T cells. Activation of the peptide-specific T cells by the MHC-binding peptide is indicated by detecting formation of the antibody-surface activation marker complex on T cells obtained from the incubation. In summary this method is similar to the ELISpot method, but attempts to resolve the accurate MHC restriction for a given peptide in the same assay. It has however added complexity compared to the pure ELISpot and still has the similar drawbacks of having to incubate cells and complications associated with readout.

WO 2004/034033 provides a system comprising a solid surface, wherein the surface has attached thereto one or more MHC monomers. The system can be used for determining binding between the immobilized MHC monomer and a putative MHC-binding peptide. The corresponding method includes the steps of incubating the solid surface having attached thereto the MHC monomers in the presence and absence of the putative MHC-binding peptide to obtain a loaded MHC monomer, and binding of a monoclonal antibody that does not bind to dissociated components of the MHC complex but only to the loaded MHC monomer, which binding indicates the binding between the putative MHC-binding peptide and the monomers. The tests can be repeated for several peptides to be tested. In this case the MHC molecules cannot be used further after the screening has been carried out for applications in solution phase, since the attachment of the molecules is normally irreversible.

WO2005/047902 discloses a method for identifying an MHC-binding peptide for an MHC monomer, said method comprising: a) incubating under suitable liquid phase conditions a sample comprising: at least one MHC monomer having bound thereto a template MHC-binding peptide, an excess amount of a first competitor peptide, and a tracer MHC-binding peptide tagged with a detectable label so as to allow competition between the first competitor peptide, the template peptide, and the tracer peptide for binding to the MHC monomer, wherein the template peptide has lower or intermediate affinity as compared with the tracer peptide for the monomer; and b) determining a difference in signal produced by the detectable label in the sample as compared with signal produced solely by monomer obtained from the sample after the incubation, wherein the difference indicates the first competitor peptide is an MHC-binding peptide for the monomer. These tests can be repeated in order to screen more than one peptide.

The methods of WO 2004/034033 and WO2005/0479 have the disadvantage that they would be difficult to carry out without purified MHC molecules or complexes as starting material. They still have a fair level of complexity and fail to offer the possibility to integrate methods of screening peptides with a wide range of binding affinities with a fast process of producing MHC-peptide complexes for those MHC binding peptides that can be used to label, detect and separate T cells in a call sample in order to identify which binding peptides are T cell epitopes.

Recently, methods of MHC class II epitope mapping, detection of autoimmune T cells and antigens, and autoimmune treatment has been described; US 2003/073102 A1. Moreover, in WO 99/21572, soluble MHC complexes and methods of use thereof have been described.

A review article focuses on tumor antigens and proteomics from the point of view of the major histocompatibility complex peptides; Admon et al. (2003), Molecular and Cellular Proteomics 2(6):1535-9476.

The direct detection of major histocompatibility complex class I binding to antigenic peptides using surface plasmon resonance has recently been described; Khilko et al. (1993), Journal of Biological Chemistry 268(21):15425-15434.

Further, a methodology for assaying the binding of a peptide to an individual, specific, soluble HLA molecule has recently been described (US 2003/124613 A1) while in WO 02/30964 a methodology for the isolation, purification and identification of peptide ligands presented by MHC positive cells is disclosed.

Moreover, in US 2004/072262 A1, it has been found that MHC heavy chain monomers immobilized to a solid surface are still capable of forming detectable conformational epitopes and being detected by conformation-dependent antibodies while methods and systems for detecting MHC class I binding peptides are disclosed.

Further, cartilage oligomeric matrix protein (COMP)-induced arthritis in rats has recently been described; Carlsén et al. (1998), Clinical and Experimental Immunology 114(3):477-484.

Moreover, a quantitative ELISA capable of determining peptide-MHC class I interaction has recently been described; Sylvester-Hyid et al. (2002), Tissue Antigens 59(4):251-258.

It is the object of the invention to provide an improvement over the prior art by providing a fast and efficient method of screening putative MHC binding peptides for binding to MHC molecules and optionally producing a set of processed (e.g. purified) MHC molecules incorporating MHC binding peptides, which method allows for high yields and lower losses in protein material.

### Summary of the Invention

The above drawbacks and disadvantages of the prior art are overcome by the present invention, which relates to the embodiments as characterized in the claims. Thus, it relates to the following items:
1. Method for screening the binding properties of constituent peptides of MHC molecules, said method comprising the steps of:
   (a) providing in solution MHC molecules or their constituent peptides for a set of MHC molecules, which set includes a plurality of subsets of MHC molecules,
      wherein the MHC molecules of each subset differ from MHC molecules of at least one other subset in at least one constituent peptide selected from the group consisting of a putative MHC binding peptide, an MHC alpha chain and an MHC beta chain, and
      loading said MHC molecules with an MHC binding peptide by
      (i) refolding of the MHC alpha chain and beta chain peptides in presence of said MHC binding peptide or
      (ii) by peptide exchange or loading with an unlabelled MHC binding peptide in the absence of any labelled MHC binding peptide,
   (b) taking of at least one sample from each subset, and
   (c) determining loading efficiency for the sample of step (b), wherein said loading efficiency is determined by measuring the proportion of loaded MHC molecules to unloaded MHC molecules,
   wherein said MHC molecules are MHC oligomers and wherein oligomerisation occurs before step b) such as after step a) or in parallel to loading by refolding in step a).
2. The method of item 1 for screening putative MHC binding peptides, wherein the MHC molecules of each subset differ with regard to the putative MHC binding peptide to be loaded in the MHC binding groove, said peptide preferably being substantially homogeneous for each subset.
3. The method of item 1 or 2, wherein in one subset a standard for the MHC constituent peptide to be screened is used and wherein the method further comprises a step of
   (d) comparing the loading efficiency of any subset to be screened to the loading efficiency of the subset including the standard.
4. The method of any preceding item, wherein loading efficiency is determined by a conformational binding assay using at least one monoclonal antibody not capable of binding to the non-loaded MHC molecule, but capable of binding to the loaded MHC molecule to form an MHC-antibody complex.
5. The method of item 4, wherein the monoclonal antibody is immobilized on a solid support.
6. The method of any preceding item which is a cell-free assay.
7. The method of any preceding item for screening the binding efficiencies of putative MHC binding peptides to MHC molecules present in a crude, unpurified mixture.
8. The method of item 7, wherein the MHC molecules are oligomers and wherein each oligomer comprises at least two chimeric proteins comprising a first section derived from an MHC constituent peptide or functional part there of and a second section comprising an oligomer forming coiled-coil domain, wherein formation of the oligomeric MHC molecule occurs by oligomerisation at the oligomerising domain of the chimeric proteins and wherein at least two of the first section of all chimeric proteins comprised in said oligomer are derived from the same MHC peptide chain.
9. The method of item 8 wherein the oligomerising domain comprised in at least on of the chimeric proteins is derived from the pentamerisation domain of cartilage oligomeric matrix protein (COMP).
10. The method of any preceding item wherein the MHC molecules contain a recognition sequence for a biotinylating enzyme fused to at least one of their constituent peptides.
11. The method of any of items 8 or 10, wherein the MHC molecules are tetramers.
12. The method of any of item 8 to 10, wherein the MHC molecules are pentamers.
13. The method of any preceding item further comprising the step of providing a set of screened MHC molecules.

It is disclosed that the method may further comprise one or more of the steps of
(e) selecting specific subsets based on the loading efficiency in step (c) or the comparison of step (d),
(f) purifying the MHC molecules in at least one of the subsets,
(g) concentrating the MHC molecules in at least one of the subsets,
(h) subjecting the MHC molecules in at least one of the subsets to a quality control to a quality control, and
(j) subjecting the MHC molecules in at least one of the subsets to one or more steps selected from a labelling reaction, lyophilisation, immobilisation on a solid surface, incorporation into a lipid (bi)layer, oligomerisation, or binding to a multivalent entity, and
(k) subjecting the MHC molecules or samples thereof to at least one of an on rate and an off rate study for quantitative determination of binding affinity of the putative MHC binding peptide to the MHC molecules in the subset,
which steps (e) to (k) may occur in any order after step (a) and may be carried out before or after carrying out steps (b), (c) and (d), if applicable.

Less than one in ten subsets may be selected in step (e) and most preferably less than one in twenty five subsets are selected in step (e).

In a preferred embodiment of the method of the invention the loading efficiency is determined by a conformational binding assay using at least one monoclonal antibody not capable of binding to the non-loaded MHC molecule, but capable of binding to the loaded MHC molecule to form an MHC-antibody complex, and optionally using a second antibody capable of binding the MHC-antibody complex, wherein optionally one of the first and second antibody is immobilized on a solid support.

Most preferably the screening method of the invention is a cell-free assay.

The method as disclosed herein preferably includes the step of purifying the MHC molecules, which purification most preferably comprises the step purifying substantially in parallel the subsets of MHC molecules as obtained in step a), e.g. by carrying out steps f1 to f3 (described later herein) substantially in parallel for individual subsets. Purification maybe by a chromatographic method, preferably using a stepped gradient for elution.

In this context "Loading" of the MHC molecules means the assembly of the constituent peptides in the molecule, including their alpha chain, beta chain and MHC binding peptide to form ternary complexes that are substantially stable.

There is also disclosed a method for identifying T cell epitopes from a set of candidate T cell epitopes which candidate T cell epitopes are identified from a set of putative MHC binding peptides through the screening method of the invention.

Preferably the method for identifying T cell epitopes comprises the step of confirming said T cell epitopes by labelling and detecting antigen specific T cells in a cell sample that have an antigen receptor that specifically reacts to the peptide sequence of a candidate T cell epitope identified according to a method as disclosed herein.

There is also disclosed the using of at least one subset of MHC molecules obtained from the method of the invention to confirm the relevance of a T cell epitope by labelling, detecting and/or separating antigen-specific T cells.

There is also disclosed a kit comprising
- a set of MHC molecules including a plurality of subsets of MHC molecules, each subset comprising at least two constituent peptides selected from the group consisting of an MHC alpha chain, an MHC beta chain and an MHC binding peptide or putative MHC binding peptide,
- at least a first monoclonal antibody capable of binding to a loaded MHC molecule, but not capable of binding to the non-loaded MHC molecule, to form an MHC antibody complex, which first monoclonal antibody is attached to a solid support, and
- a second antibody capable of binding the complex of loaded MHC molecule and first antibody.

### Detailed Description of the Invention

As described above, the drawbacks and disadvantages of the prior art are overcome by a method of screening a set of MHC molecules and optionally providing the screened MHC molecule subsets in purified and or otherwise processed form.

The method of the invention - by screening and processing of a plurality of subsets in parallel - allows for rapid and economic method of screening of large sets or entire libraries of MHC molecules, preferably for MHC binding peptides, which screening can be carried out before, during or after purification of the assembled MHC molecules. At the same time this method is useful for producing functional MHC molecules such as MHC oligomers for detecting labelling and separating antigen specific T cells from the same material that the screening is performed on, i.e. it is not necessary to set up the MHC oligomer synthesis as a separate process from the screening step. If the screening is carried out after the assembly of the loaded MHC molecules, but in advance of the purification process, it allows for the benefit of avoiding purification efforts for MHC complexes that have not assembled sufficiently to be functionally useful. The method of the invention further allows for rapid small-scale production of screened MHC complexes.

A loaded MHC molecule or MHC complex comprises an alpha chain peptide, a beta chain peptide and an MHC binding peptide bound in the groove of the molecule. The binding efficiency of these three components and especially of the MHC binding peptide is crucial for effectiveness of the loaded MHC molecule in activating a T cell of the corresponding antigen specificity. This loading efficiency will typically be determined by measuring the proportion of loaded MHC molecules to unloaded MHC molecules following the loading reaction. Screening of the loading efficiency is therefore a reasonable measure or indicator of the effect the loaded complex may have on a corresponding T cell.

Since the loading efficiency will depend on the mutual binding affinity of all three components constituting the MHC molecule, the method of the invention can thus in principle be exploited to screen not only for the affinity of the MHC binding peptide, but for any of the three constituent peptides of said complex, where to respective other two are then kept constant. For alpha chain peptides or beta chain peptides the method of the invention thus e.g. allows for screening on the influence of mutations in the MHC alpha or beta chains or the influence of added sequence parts or oligomerisation of the MHC molecule on binding efficiency of a desired peptide.

Preferably, however, screening is carried out on the MHC binding peptide. This means that preferably the MHC molecules of each subset differ with regard to the MHC binding peptide bound in the MHC binding groove, said peptide preferably being substantially homogeneous for each subset. Due to several subsets being processed in parallel, which subsets include differing MHG binding peptides, the method of the invention allows for parallel screening of a plurality of peptides, and even a full library of peptides at the same time. This library could e.g. be derived from a single (antigenic) protein for a certain type of T cell to find possible epitopes for such antigen specific T cell. Peptides could be derived from the protein by creating overlapping fragments of a certain length thereof. While the screening method of the invention can be used as a stand-alone method it can, if desired, still be combined with screening methods of the prior art. For example, if the set of peptides to be screened is very large, e.g. 10⁴ to 10⁶, which may be the case if whole section of a pathogen proteome is to screened the cost of peptide synthesis will be considerable. In this case pre-screening with an algorithm, such as SYFPEITHI may still be a good option to reduce the number of peptides selected for actual screening by one to three orders of magnitude.

In a preferred embodiment one or more standards of the MHC complex peptide to be screened, and preferably the MHC binding peptide, is used in one subset and the method further comprises a step of (d) comparing the loading efficiency of the subset to be screened to the loading efficiency of the subset(s) including the standard(s). If for example the natural epitope of a T cell is used as the standard MHC binding peptide, the comparison of loading efficiency of all tested peptides with the same will reveal relative improvements and deterioration of the tested peptide of each subset with regard to the efficiency of the natural epitope peptide.

Where the determining loading efficiency is used as a measure to select sub-sets of MHC molecules for further processing the cut-off threshold will depend on the nature of the project.

A standard may be chosen from a range of intermediate or borderline affinity binding epitopes for a given MHC allele so that subsets that are loaded more efficiently than the standard(s) and those that are loaded with less efficiency than the standards can be distinguished as good and poor candidate T cell epitopes, respectively.

Where the method may further comprise one or more of the aforementioned steps of (e) to (k), preferably at least steps (e) and (f), and most preferably steps (e) to (k) are carried out after steps (a) through (d). Preferably in the above method step (e) occurs after step (a), (b) and (c) and subsets are selected in step (e) before further processing by subjecting the selected subsets to at least one of steps (f)-(k).

The product of step a) may be subjected to step f) without prior concentration. The advantage in this case is that protein concentration losses are avoided.

By selecting those subsets of MHC molecules to whom the candidate peptides in the screening step have bound to with reasonable efficiency before the respective subsets are processed further, the method of the invention increases effectiveness of producing functional MHC molecules for a set of putative T cell epitopes as it avoids further processing subsets of MHC molecules that are unlikely to be functional or present relevant T cell epitopes.

In a preferred embodiment of the method of the invention the loading efficiency is determined by a conformational binding assay using at least one conformational antibody capable of binding to the loaded MHC molecule, but not capable of binding to the monoclonal MHC molecule, to form an MHC antibody complex, and optionally using a second antibody (preferably monoclonal) or other reagent capable of binding the complex, wherein optionally one of the conformational antibody and the second antibody/detection reagent is immobilized on a solid support. Examples for conformational monoclonal antibodies against MHC molecules include the following clones:
W6/32 (ATCC No. HB-95) and MEM123 (supplied by Abcam Limited, Cambridge, UK cat. no. ab2217) both recognize human MHC class I alleles;
BB7.2 specifically recognizes HLA-A2 (ATCC No. HB-82);
BB7.1 specifically recognizes HLA-B7 (ATCC No. HB-56); and
L243 f specifically recognizes HLA-DR (ATCC No. HB-55).

The antibody/detection reagent that is not immobilized on the solid support may be provided with a detectable label, i.e. a label that produces a detectable signal as is known in the art. Labels may be incorporated or conjugated according to any known method. Suitable labels may include without limitation fluorochromes, enzymes, biotin, radioisotopes.

More preferably the conformational antibody is immobilized on a solid support and is thereby used simultaneously for conformational probing and as a capture reagent. Since in this case only conformational material is bound on the solid support (e.g. a microtiter plate, resin, bead or the like) from a mixture of conformational and non-conformational material in a given subset of MHC molecules this improves the background signal and signal to noise ratio of the assay. This method is therefore particularly suitable for screening the binding efficiencies of putative MHC binding peptides to MHC molecules present in a crude, unpurified mixture, such as refolding solution that comprises an excess of non-conformational or aggregated MHC alpha and beta chains compared to correctly refolded material or tissue culture supernatant into which the MHC molecules may have been expressed, e.g. through soluble expression in an eukaryotic cell line, such as insect or mammalian cells. A second detection antibody or other detection reagent will then typically be used to detect the conformational MHC molecules. The detection antibody or other reagent may comprise a detectable label or a detectable binding domain.

In a preferred embodiment where the conformational antibody is used as a capture reagent the binding assay is carried out by binding a standard in competition with a sample of MHC to be evaluated in the same assay. In this case the MHC molecules constituting the standard may have a detectable label attached and the formation of conformational material in the sample is detected by measuring the inhibition of binding caused by the sample versus binding of the standard.

Preferably, the second antibody includes or has attached thereto a detectable marker known in the art. For example, any of the following markers can be used: Fluorescent, chemiluminescent, radioactive and enzyme markers such as alkaline phosphatase, (horseradish) peroxidase or biotin can be used. Fluorescent and enzyme markers are the preferred markers in this embodiment of the invention.

Most preferably the screening method of the invention to determine MHC-peptide binding is a cell-free assay (T cell free assay). This means that the screening does not require the presence or inclusion of any cells in the screening as such. This does not exclude a confirming of the MHC binding peptides identified by the screening method of the present invention as T cell epitopes through binding of the corresponding MHC-peptide molecules to a cell sample comprising the respective antigen-specific T cells.

Preferably the set of putative MHC binding peptides to be screened is derived from the same protein or polypeptide.

Usually when one studies many different proteins in an experimental setting and the study requires purification, such different protein species will have quite different physical properties and therefore have to be purified by different purification methods which may include a variety of chromatography methods, such as size exclusion chromatography, anion and cation exchange chromatography, hydrophobic interaction chromatography, affinity chromatography. At least such proteins will usually be eluted under different conditions using the same purification method due to the difference in their physical properties. This is different for the MHC molecules of the subsets, which should substantially have the same physicochemical characteristics.

Purification of the subsets of MHC molecules obtained from step (a) may be further purified to provide the molecules for further use e.g. in research, diagnostic or therapeutic applications. Purification can in principle be carried out using any conventional method known in the art.

Standard production of monomeric biotinylated MHC peptide complexes as described by the NIAID tetramer core facility as available on 31 March 2005 under the following URL: http://www.yerkes.emory.edu/TETRAMER/pdf/Protocols.pdf can be applied. It typically involves refolding of denatured MHC alpha and beta chains of the desired MHC allele in the presence the desired MHC binding peptide in a 500ml volume of refolding mixture, followed by concentration of the refold mixture on a stirred cell concentrator to approximately 7ml. The alpha chain typically has an extension at its c-terminus which incorporates a biotinylation signal sequence that can be biotinylated with a biotinylating enzyme. This will be followed by buffer exchange into biotinylation buffer using a gravity driven desalting column. Biotinylation is carried out in the presence biotin and a suitable biotinylating enzyme, e.g. BirA. Purification is done first by size exclusion chromatography which requires approximately 90 minutes run time and further time for regeneration of the system. Biotinylated monomers are then optionally concentrated again in a centrifugal filtering device before loading onto an ion exchange chromatography, column for final purification.

Preferably, however, a parallel purification methods as disclosed in a patent publication GB 2424645 B is adopted. This method is based on the idea that various MHC molecules, though-differing slightly e.g. in the peptide, are still usually very similar in their over all physical properties such as their isoelectric point, molecular weight, conformation and hydrophobicity. This allows their parallel purification by applying essentially the same or very similar methods and conditions. Parallel processing reduces manufacturing and hence product costs.

In the context of this disclosure the term "substantially in parallel" means that the individual subsets are subjected to the same or similar process steps in close timely relationship, and preferably at substantially the same time or simultaneously. This does not require all of the subsets being processed at the same time, but is intended to include scenarios, wherein e.g. a first group of subsets is processed first and a second group is processed thereafter, and so on. Preferably, in parallel means a simultaneous processing of all subsets, even more preferably under similar or identical conditions. Parallel processing may e.g. be in the same device (such as a centrifuge or a vacuum manifold) or by using parallel-operated equipment.

Substantially parallel processing will preferably occur especially in step f), i.e. during purification. To meet this requirement at least one of the purification steps of loading on an adsorber, washing and elution is carried out in parallel. Typically at least elution/recovery is carried out in parallel. More preferably, at least washing and elution are parallel.

Providing the MHC molecules for each subset or loading of MHC molecules with the putative MHC binding peptide of step a) needs not be parallel for each subset, but can of course be in parallel, if technically feasible and/or desirable.

Since the MHC molecules will be provided in solution in step a) the type of chromatography will usually be a liquid chromatography.

The chromatographic method of step f) typically involves the steps of
(i) loading by contacting the surface of a chromatography adsorber matrix with a mixture of molecules in liquid solution, which solution includes a specific type of molecule to be purified under conditions so that at least the molecules to be purified will bind to the absorber matrix,
(ii) optionally washing, if desired or necessary to achieve the intended purity, and
(iii) after such binding has occurred the step of elution by changing the composition of the buffer in which the chromatograpy matrix is immersed over time (whether continuously or in one or more steps) until the molecules to be purified elute from the chromatography matrix.

Elution may be by any appropriate means or gradient as desired and known in the art. Preferably, elution is by using a stepped gradient as described in patent publication GB2424645 B. Using a stepped gradient for elution shall within the scope of the invention mean changing the composition or property of the solution in which the chromatography matrix is immersed in one or more discrete steps as opposed to changing it continuously. For the purpose of distinguishing a continuous gradient from a stepped gradient, a continuous gradient may for example be created by analogous operation of a mixing valve or quasi-continuous change in mixing of two or more solvent components under digital computer control, such as in a computer operated liquid chromatography system. Hence, using a stepped gradient for elution will usually involve preparing separate aliquots of solutions with different properties (such as differing salt concentration or pH), which may be applied in a step-wise sequence one after another until elution occurs. The number of steps in which this gradient is applied will depend on the chromatographic method chosen, tolerable losses and the desired purity. The stepped gradient will at least include one step and typically less than 50 steps, preferably less than 10 steps, more preferably less than 2 steps and most preferably only one step.

The MHC molecule subsets differ from at least one, and preferably any other subset in at least one element selected from the group consisting of the putative MHC binding peptide to be bound in the MHC binding groove, an MHC alpha chain and an MHC beta chain. The differing subsets taken together comprise the set to be provided by the present invention. Each subset may be processed in an individual batch. This limitation does not exclude the possibility of individual subsets being present in more than one batch, provided that the set includes at least two differing subsets.

The set as produced may comprise at least 2, preferably at least 24 and more preferably at least 96 subsets of MHC molecules. The number of subsets may depend on the precise screening task, and e.g. the number of peptide fragments of a potential antigen created. It may also depend, incase later purification is desired, on the method of chromatography chosen and the device used for implementing the same.

Preferably, the chromatographic method is one using an adsorber matrix selected from the group consisting of resins, beads, and membranes (such as a porous membrane adsorber chromatography matrix). Column chromatography methods are preferred over batch designs, due to ease of handling and availability of equipment. Various adsorption types of binding properties may be exploited such that the method may be chosen from the group consisting of affinity chromatography, ion exchange chromatography, iso-electric focussing chromatography, hydrophobic interaction chromatography, hydroxyl-apatite chromatography, and reverse phase chromatography; ion exchange chromatography being most preferred. In one preferred embodiment the adsorber is a porous membrane adsorption matrix, such as described in WO/0119483. More preferably such matrix is an ion exchange chromatography matrix.

Ion exchange chromatography most preferably uses a basic ion exchanger such as a resin comprising quaternary ammonium groups (DEAE). Commercial ion exchange resins suitable for use in the present invention are e.g. those sold under the trademarks Sepharose®, MonoBeads® from GE Healthcare (formerly Amersham Biosciences).

Preferably, the purification step f) comprises the steps of
f1) loading the MHC molecules in at least one subset obtained in step a) on an adsorber matrix, preferably in form of a column,
f2) washing the loaded adsorber matrix at least once,
f3) eluting the adsorber matrix by applying a stepped gradient,
f4) recovering the MHC molecules in the eluate of step f3), and
f5) optionally repeating steps f1 to f4, individually or in combination, until the desired purity of the MHC molecules is obtained.

Typically the method of the invention will yield a purified set of MHC molecules, wherein impurities are removed from the MHC molecules in each subset by in the wash step(s) before elution the matrix before elution and/or remaining bound to the matrix after the elution step(s). The impurities that typically remain on the column after the elution step(s) are usually larger protein aggregates. The method of the invention also allows for efficient buffer exchange of the MHC molecules in each subset from the buffer that they are provided in step a) to the buffer they are eluted in.

The stepped gradient used in the chromatographic method is preferably any suitable gradient depending on the chromatographic method chosen. It will typically be selected from the group consisting of a salt gradient, a pH gradient or a gradient employing a a denaturing or chaothrophic agent, and mixtures thereof. Most preferably the stepped gradient is a one-step gradient such as a one-step salt gradient.

The concentrations of the gradient are likewise chosen depending on the chromatographic method chosen and adsorption strength. For ion exchange chromatography preferably a salt gradient of at least 100 mM salt, preferably 200 to 500 mM salt is used. Most preferably the salt is NaCl.

The product of step a) is loaded on the adsorber-in the following reference will be made to columns only, though it is to be understood that the same or comparable conditions will apply to other types of adsorbers as well - under any suitable conditions as to capacity, number of loading steps, buffer, flow rate, pH and temperature.

The column is then subjected to washing and elution. The solution used for at least one of the steps of purification selected from loading, washing and elution is any suitable solution such as an aqueous solution of pH above 7, preferably above 7.5. Identical solutions are typically chosen for the wash buffer and the loading buffer, but need not be. Elution occurs by using the stepped gradient as above. The entire sequence of purification steps in f) (f1 to f4) may be repeated individually (e.g. more than one washing step applied) or in combinations (sequence f1 to f4 is repeated), as desired.

The purified material may optionally be buffer exchanged into a suitable storage buffer, which may contain stabilizers such as BSA, preservatives, such as sodium azide and protease inhibitors.

Preferably, the conditions for carrying out at least one of the steps of purification in f) are substantially identical for at least two subsets and more preferably for all subsets.

In one embodiment the MHC molecules of each subset are provided and loaded with the desired peptide to be screened in step a) by refolding MHC alpha and beta chains of said MHC molecules in the presence such peptide as known in the art, e.g. by following a protocol described in Garboci et al., PNAS 89 (1992), 3429-3433 or the NIAID tetramer core facility protocol *(supra).* In both cases the MHC α and β chains are expressed in a bacterial host and obtained from inclusion body material.

In an alternative embodiment the MHC molecules of each subset are loaded in step a) by exchanging the peptide bound in said MHC molecules under suitable conditions to load the desired peptide as known in the art, such as disclosed in WO9310220 and T.O. Cameron et al., J. Immunol. Meth. 268 (2002), 51-69. In this case the MHC molecules for each subset are obtained by expression in a eukaryotic host and they may contain no peptide, peptide endogenous to the host cell or other irrelevant peptide before loading of the desired peptide of interest for each subset. The exchanged peptide is unlabelled and peptide exchange also occurs in the absence of any labelled peptide. Preferably, only the peptide initially bound in the MHC molecule's binding grooves and the unlabelled MHC binding peptide to be screened and exchanged therefore are present as potential peptides in this exchange reaction. Some MHC alleles, especially Class II MHC alleles can also be stable in the absence of a binding peptide, i.e. they can form unloaded heterodimers of their respective alpha and beta chains. In such a case loading of the putative MHC binding peptide may not effect a significant conformational change of the loaded MHC molecule versus its unloaded state. In this case a good binding MHC peptide should still be able to improve the stability of the MHC molecule compared to the unloaded state. Slightly denaturing conditions, such as high temperatures, low or high pH or chaotropes may be applied in this case to bring the MHC molecule to a state where a difference between the loaded and unloaded conformation of the molecules is detectable with the methods described herein. In the event that a peptide exchange is carried out in the method of the invention with an MHC molecule that is stable empty it maybe desirable to use a conformational antibody in the binding assay that not only detects the conformation of the MHC molecule, but also detects such conformation only in the presence of a specific starting peptide present in the molecule.

Hence depending on the embodiment chosen, providing the MHC molecules for each subset and loading with the said peptide of interest may take place simultaneously or in sequence.

As disclosed herein, the MHC molecules maybe selected from MHC monomers and oligomers. The term oligomer is intended to include any molecule comprising more than one MHC monomer, each monomer consisting of alpha chain, beta chain and peptide bound in the binding groove. In a preferred embodiment the oligomers are formed by joining the MHC monomers through a suitable known method in the art. Examples of such oligomers are dimers to decamers, especially dimers, tetramers, pentamers and decamers. Oligomerisation may occur as disclosed in the above references or in WO93/10220 WO2004/018520. In a preferred embodiment the MHC molecule is a tetramer. More preferably it is a tetramer obtained by joining MHC monomers through the biotin/avidin, biotin/streptavidin or Streptag®/Strep-tactin® binding pair. Streptag® and Strep-tactin® are described in US5,506,121 and US6,103,493, respectively and reagents incorporating both technologies are available from IBA GmbH, Goettingen, Germany. In an alternative embodiment, the MHC molecule is a pentamer.

Preferably the MHC molecules produced in the method of the invention are oligomers and oligomerisation can occurs at any time, e.g. before or after purification. Preferably oligomerisation will occur before step f) and more preferably before step b), such as during or after step a).
The screening method according to the invention can thus be carried out by loading the molecules through refolding or peptide exchange on monomeric MHC molecules or oligomeric MHC molecules, such as MHC tetramers or pentarners, which has the advantage that no additional oligomerisation step may be required in order to produce functional MHC oligomers for labelling, detecting and separating antigen specific T cells, after the peptide loading has taken place. In this case the oligomer is an oligomer of at least one of the MHC alpha or beta chains.

The peptide exchange in a heterodimeric MHC molecule comprising its alpha and beta chain can be carried for example out as described in WO93/10220 or in WO2005/047902. Typically the desired putative MHC binding peptide will be incubated with MHC molecules in a suitable exchange buffer, e.g. at elevated temperature such as 30°C or 37°C to accelerate the exchange. The MHC molecules provided for the exchange will preferably have an placeholder MHC. binding peptide bound which only has a low binding affinity. The exchange buffer may adjusted so that the conformation of the MHC molecules is opened slightly through by having a relatively low or high pH, such as pH 2-5.5 or pH 9-11 or added chaotropic agents urea or guanadinum hydrochloride.

In a preferred embodiment the MHC molecules produced in the method of the invention are oligomers wherein each oligomer comprises at least two chimeric proteins comprising a first section derived from an MHC constituent peptide or functional part there of and a second section comprising an oligomer forming coiled-coil domain, wherein formation of the oligomeric MHC molecule occurs by oligomerisation at the oligomerising domain of the chimeric proteins and wherein at least two of the first section of all chimeric proteins comprised in said oligomer are derived from the same MHC peptide chain.

Such MHC molecules are described in WO2004/0185 e.g. an oligomeric MHC complex wherein either the MHC alpha or beta chain are fused to an oligomerising domain, wherein preferably the oligomerising domain is derived from an oligomer-forming coiled-coil protein and wherein more preferably formation of the oligomeric MHC molecule occurs by oligomerisation at the oligomerising domain of the chimeric proteins.

Preferably the first section of the chimeric protein in the oligomeric MHC molecules is derived from the extra-cellular part of the MHC class I or class II alpha or beta chain.

More preferably the oligomerising domain comprised in at least on of the chimeric proteins is derived from the pentamerisation domain of cartilage oligomeric matrix protein (COMP). Most preferably this domain can be fused to one of the termini of beta 2 microglobulin in the case of class I MHC or the beta chain of class II MHC.

Most preferably the pentamerisation domain of COMP comprised in the second section in at least on of the chimeric proteins comprise and preferably consists of the amino acids 1 to 128, preferably 20-83 and most preferably 20-72 of COMP.

It is also disclosed that the MHC molecules may be MHC monomers, which have been biotinylated, preferably before subjecting them to step f). Preferably, the MHC monomers have been biotinylated on either their alpha or beta chain even before loading of the peptide either by refolding or peptide exchange in step a). Biotinylation of the MHC monomers can be achieved as known in the art, e.g. by attaching biotin to a specific attachment site which is the recognition site of a biotinylating enzyme. Reference is in this regard made e.g. to EP 812 331. Preferably, the MHC molecules contain a recognition sequence for a biotinylating enzyme fused to at least one of their constituent peptides and are optionally biotinylated at such recognition sequence. Preferably the biotinlylating enzyme is BirA. In a preferred embodiment, biotinylation is carried out on the desired protein chain *in vivo* as a post translational modification during protein expression of such protein chain in the expressing host cells as described in WO/9504069.

In case the MHC molecules are desired as MHC oligomers the method of the invention further includes the step ofoligomerizing the MHC monomers to yield the desired oligomer. Such step of oligomerization may occur after purification. Alternatively it may occur before purification, either before step f) and after step a), or alternatively before or during step a).

The method may further include a step of purifying the α and β chains of the MHC molecules before providing the MHC molecules in step a). Purification of the α and β chains as such or aligned to an MHC molecule may be carried out by any conventional method and preferably by ion exchange or affinity chromatography.

The driving force for at least one of the purification steps selected from loading, washing and elution is typically selected as appropriate for the method and device of chromatography chosen. It is preferably selected from gravitational forces, a centrifugal force applied by centrifuging the chromatography column or a pressure drop caused through applying a vacuum to one end of the column.

Preferably, the plurality of subsets is purified substantially simultaneously in the same centrifuge or on a vacuum manifold. Corresponding vacuum manifolds are known in the art and are commercially available e.g. from Qiagen GmbH, Hilden, Germany, under the tradename QIAvac 24 plus, which is a 24 place manifold. Alternatively, a commercially available centrifuge is used for spinning of appropriate columns.

Especially for processing of very small-scale samples it is preferred that the chromatography column is mounted directly on the receptacle receiving the eluate during the elution step f4). Corresponding spinning columns are commercially available e.g. from Vivascience AG, Hannover, Germany, under the trademark VivaPure® such as the VivaPure® Q Mini H column. The receptacle may also be a microcentrifuge tube of between 0.5 to 2 ml volume such as those known in the art as Eppendorf® tubes.

Preferably no concentration steps are carried out before subjecting the product obtained from step a), i.e. the peptide loaded MHC molecule (monomer or oligomer) to the chromatography of the step f), and especially before loading the product of step a) onto the adsorber. More preferably, none of a separate concentration or separate buffer exchange step is carried out between step a) and step f). In other words, the product of step a) is eventually subjected to step f) essentially as is or after an oligomerisation thereof.

The product obtained in step b) may further be subjected to one or more steps selected from a labelling reaction, lyophilisation, immobilisation on a solid surface or incorporation into a lipid (bi)layer or quality control e.g. by SDS-PAGE, immune precipitation or any other analysis method, such as a conformational binding assay. Any of these steps may likewise be carried out in parallel for the subsets, but need not be so.

In order to determine the binding affinity of a peptide more specifically, the on rate and/or off rate of an MHC-binding peptide can optionally be determined. An approximation of the on rate *in vivo* can be determined by taking samples of the refolding or loading mixture in each subset at several time points in the reaction and measuring the level of loading versus a fully assembled standard sample with the same or similar conformational binding assay for determining loading as described hereinbefore. The time points may e.g. be equally spaced over the loading period or be taken at doubling times. The data for the time points can be used to create an association curve, which can be calculated to calculate the on-rate or association-rate according to standard methods known in the art

Equally the off-rate or dissociation rate of the peptide can be calculated by measuring the stability of assembled and preferably purified MHC molecules at a variety of elevated temperatures at different time point. Taking measurements at 37°C is the closest approximation for the off-rate *in vivo* and, as is mentioned in WO2004/034033, typical half-lived of immunodominant peptides are greater than 20 hours at 37°C. Specifically the MHC molecules loaded with peptides of interest will be incubated at the desired temperature, e.g. for 48 hours, taking off samples at 0, 4, 12, 24, and 48 hours and measuring the loading of the complexes by conformational binding assay against a standard as described hereinbefore. A dissociation curve can be established by interpolating the data and the half-life and off-rate can be calculated from this by standard methods known in the art

The on rate experiment can be done, e.g. on all candidate MHC peptides during loading or only on a selection of peptides in a repeat reaction after the first loading step has been completed and first data on loading efficiency has been obtained. In this case for example, only promising candidate MHC binding peptides may be studied for on-rate in a repeat reaction of the loading procedure.
Similarly the off rate analysis would typically be carried out on purified MHC peptide complexes, e.g. only for those complexes that have been chosen for further processing.

As is apparent from the above, the method of the present invention is especially suited to be carried out in small scale. With small-scale batches of 200 µl to 500 ml per subset, preferably 1 to 10 ml per subset are meant. These can be handled in the above mentioned equipment which itself is commercially available.

The MHC molecules are selected from the group consisting of MHC class I, MHC class II, non-classical MHC, CD1, homo-oligomers thereof, hetero oligomers thereof and mixtures of the same. The MHC proteins may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2-microglobulin. Included in the murine H-2 subunits are the class I H-2K, H-2D, H-2L, and the class II I-Aα, I-Aβ, I-Eα and I-Eβ, and β2-microglobulin. Amino acid sequences of some representative MHC proteins are referenced in EP 812 331. Also included in the scope of this invention are non-classical examples such as HLA-E, HLA-F, HLA-G, Qa1, and CD1. The CD1 monomer may instead of the peptide have a lipid bound in its groove. The present invention is also applicable to the situation where a lipid instead of a peptide is bound and the skilled worker will be capable of translating the above protocols to this situation.

The MHC peptide chains may correspond to the soluble form of the normally membrane-bound protein. For class I subunits, the soluble form is derived from the native form by deletion of the transmembrane and cytoplasmic domains. For class I proteins, the soluble form will include the alpha1, alpha2 and alpha3 domains of the alpha chain and beta 2 microglobulin. For class II proteins the soluble form will include the alpha1 and alpha2 or beta1 and beta2 domains of the alpha chain or beta chain, respectively.

In general, the MHC molecule may in one or more of the proteins or peptide chains comprised therein further comprise one or more additional domains such as one or more linkers, a tagging domain and a purification domain. The additional domain(s) may e.g. be provided on the multivalent entity in case of oligomers or on the peptide, the MHC alpha and/or beta chains, respectively.

The screening method of the invention may finally comprise the step of providing a set of screened, and more preferably screened and selected subsets of MHC molecules.

As mentioned before, there is also disclosed a kit for screening MHC molecules.

Preferably the kit further comprises a plurality of containers each including a subset of MHC molecules. More preferably, the plurality of containers is provided in form of a plurality of spinning columns as described above or a microtiter plate.

Preferably each subset comprises an MHC alpha chain and an MHC beta chain for forming empty MHC molecules, and wherein preferably the empty MHC molecules of preferably subset are identical.

In the kit one of the first or second antibody may be immobilized on the container wall. One of the first and second antibody may have attached thereto a marker for detection thereof. Said marker can be any one of the markers and labels described above.

The kit as disclosed herein may further comprise one or more elements selected from the group consisting of an antibody, a buffer, a label, an enzyme, an enzyme substrate, a standard, an instruction leaflet, a microliter plate which may have reagents pre-immobilized, and a T cell sample.

In general the kit may contain additionally contain any other substance or item described herein for use in carrying out the method of the invention.

The present invention will be further illustrated by way of the following examples, which is however not intended to limit the same.

### Example 1

In this example a set of 500 different subsets ofbinding peptides, which may e.g. be derived as overlapping 9-mer peptides from a 509 amino acid protein offset from one another by one amino acid each, are screened for binding to biotinylated monomeric MHC I molecules and a smaller set of MHC molecules is selected for further purification after screening and purified in a method according to the invention.

**Step a):** 2 ml small scale refolds of soluble HLA-A*0201 molecules with each of the sub-sets of binding peptides are set up according to the protocol as described in Garboci *et al. (supra),* by scaling down the refold method described therein to the volume of interest. Solubilised HLA-A*0201 alpha and beta 2 microglobulin were obtained as described in the reference with the modification that the alpha chain was obtained in a pre-biotinylated form following the protocol described in WO95/04069 by fusing a biotinylation peptide to the C-terminal end of the alpha chain. Where this protocol uses stirring of the refolding mixture, vortexing is used following preparation of the refolding mixture and after the addition of each of the protein chains and the binding peptide. Refolds are incubated overnight at 4°C.

**Step b):** Samples of 10 µl are taken from each batch obtained in step a) above. **Steps c) + d):** Loading efficiency of the MHC binding peptides is determined using a sandwich ELISA wherein a first monoclonal antibody BB7.2 is bound to the wells of a 96 well microtiter plate at 100ng/well. The plate is incubated for 1 hour at 4°C. After binding of the antibody the plate is washed and blocked with 200µl of 5% BSA in phosphate buffered saline (PBS), incubated for 1 hour at 4°C. The samples, are diluted 1 in 50 and added to the wells in at 100µl per well. Samples are added to further wells at 6 doubling dilutions. The plates are then incubated at room temperature for 1 hour. Thereafter the plates are washed three times with PBS. Thereafter 25ng streptavidin-HRP conjugate diluted in 100µl per well is added and incubated for 1 hour at room temperature. The plates are then washed three times with PBS. Thereafter 50 µl/ well of tetramethylbenzedine (TMB) substrate solution is added to the wells and incubated for 7.5 minutes at room temperature. The reaction is then stopped with 50 µl per well of 0.5 molar H₂SO₄. Absorbance readings are then taken with a Tecan GENios plate reader at a wavelength of 450 nanometers.

This procedure is carried out substantially simultaneously with all the samples and with a standard which in this case is an EAAGIGILTV (single letter amino acid description), a HLA-A*0201 binding peptide with intermediate binding affinity.

**Step e):** Based on the loading efficiency as determined is step c), individual batches obtained in step a) are selected to exhibit similar or better signals than the control peptide. Selected complexes are processed further in step f).

**Step f):** The selected batches are then subjected to the following purification protocol: All batches are filtered through a 0.22µm syringe filter prior to purification using VivaPure® Q Mini H anion exchange columns. Purification is carried out by applying centrifugal force in a microcentrifuge with a 24 place rotor accepting 2ml Eppendorf® microcentrifuge tubes. Specifically, the following steps were applied:
a) Equilibration of each column with 400 µl loading buffer (20mM Tris/HCL pH 8.0) and centrifuge for 5 min at 2000g in a minifuge.
b) Loading of crude refold sample of step a) into the insert (up to 400 µl, one batch per tube) and centrifuge for 5 min as above. Loadings were repeated as appropriate until all of the sample from each batch was loaded
c) Resin wash step 1: Addition of 400 µl loading buffer to insert and centrifugation for 5 min as above.
d) Resin wash step 2: Addition of 400 µl loading buffer to insert and centrifugation for 5 min as above.
e) Sample elution: Addition of 400 µl elution buffer (20mM Tris pH 8.0 + 300mM NaCl) to insert and centrifugation for 5 min as above.
f) Recovering pure MHC molecules in the elution buffer at the bottom of the tube.

Following purification, the protein amount and concentration in each sample is determined by the method of Bradford. With dilute samples the Bradford method may require protein concentration, which may be an undesirable step and alternatively the protein concentrations can be determined by quantitative densitometry on an SDS-PAGE gel, such as by using the Genetools™ software provided by Syngene, Cambridge, UK, and comparing the density of bands of the sample with the density of a band from a sample of similar molecular weight and known protein amount.

Purified biotinylated MHC monomers are now ready for coupling to streptavidin in a variety of applications. In one application they can be coupled to R-PE labelled streptavidin in a 4:1 molar ratio of biotinylated monomer to streptavidin molecule to provide MHC tetramers that can be used to detect antigen specific T cells in flow-cytometry.

### Example 2

In this example a set of 500 different subsets of binding peptides, which may e.g. be derived as overlapping 9-mer peptides from a 509 amino acid protein offset from one another by one amino acid each, are screened for binding to and formation of pentameric MHC-peptide molecules.

Beta 2 microglobulin COMP fusion proteins are cloned, expressed, refolded to form pentamers and purified as described in WO2004/018520. The biotin acceptor sequence at the C-terminal end is replaced of the molecule is replaced by a hexahistidine epitope tag sequence.

Equally HLA-A*0201. heavy chain is cloned, expressed and recovered as described in WO2004/018520.

**Step a)** 2 ml small scale refolds of soluble HLA-A*0201 pentamers molecules with 500 different binding peptides are set up according to the protocol as described in WO/2004/018520 wherein soluble HLA-A*0201 alpha chain is refolded with purified soluble beta 2 microglobulin pentamer and by scaling down the refold method described in the reference to the volume of interest. The time of refolding was extended to 24 hours.

**Step b):** Samples of 10 µl are taken from each batch obtained in step a) above.

**Steps c) + d):** Loading efficiency of the MHC binding peptides is determined with the same sandwich ELISA as in Example 1.

Equally to Example 1 his procedure is carried out substantially simultaneously with all the samples and with a standard, which in this case is an EAAGIGILTV.

This procedure is carried out substantially simultaneously with all the samples and with a standard which in this case is an EAAGIGILTV (single letter amino acid description), a HLA-A*0201 binding peptide with intermediate binding affinity such as.

**Step e):** Based on the loading efficiency as determined is step c), individual batches obtained in step a) are selected.

**Step f)** is carried out the same way as in Example 1

Following purification, the protein amount and concentration is determine according to the same methods as in Example 1

Pentameric MHC-peptide complexes are ready for detecting antigen specific T cells in flow cytometry. Cells can be stained by a first incubation with the relevant MHC pentamer followed two washes and labelling with a suitably titrated anti hexahistidine-tag monoclonal antibody that is labelled directly with the fluorochrome of choice.

In summary, compared to prior art method described in WO2004/034033 for screening MHC binding peptides the method of the invention does not rely on immobilizing MHC molecules first before the assay is carried out which makes it possible to continue to process the MHC molecules from the same batch that has have screened into purified functional molecules that can be oligomerised or are already oligomeric for labelling, detecting and separating antigen-specific T cells.

Equally compared to the prior art method described in WO2005/047902 which describes a solution phase assay, the method of the present invention does not rely on screening against a labelled tracer peptide, which simplifies the screening method and makes it possible to continue to process the MHC molecules from the same batch that has have been screened into purified functional molecules that can be oligomerised or are already oligomeric for labelling, detecting and separating antigen-specific T cells for a wide range of MHC peptides with varying binding affinities without the constraint that the exchanged peptide needs to be of significantly higher affinity than the labelled tracer peptide and the template peptide described therein. Further it is also not necessary to provide MHC molecules pre-loaded with a predetermined template peptide as the starting material for the screening, which would otherwise require for the starting material to be pre-purified.

## Claims

1. Method for screening the binding properties of constituent peptides of MHC molecules, said method comprising the steps of:
(a) providing in solution MHC molecules or their constituent peptides for a set of MHC molecules, which set includes a plurality of subsets of MHC molecules,
wherein the MHC molecules of each subset differ from MHC molecules of at least one other subset in at least one constituent peptide selected from the group consisting of a putative MHC binding peptide, an MHC alpha chain and an MHC beta chain, and
loading said MHC molecules with an MHC binding peptide by
(i) refolding of the MHC alpha chain and beta chain peptides in presence of said MHC binding peptide or
(ii) by peptide exchange or loading with an unlabelled MHC binding peptide in the absence of any labelled MHC binding peptide,
(b) taking of at least one sample from each subset, and
(c) determining loading efficiency for the sample of step (b), wherein said loading efficiency is determined by measuring the proportion of loaded MHC molecules to unloaded MHC molecules,
wherein said MHC molecules are MHC oligomers and wherein oligomerisation occurs before step b) such as after step a) or in parallel to loading by refolding in step a).

2. The method of claim 1 for screening putative MHC binding peptides, wherein the MHC molecules of each subset differ with regard to the putative MHC binding peptide to be loaded in the MHC binding groove, said peptide preferably being substantially homogeneous for each subset.

3. The method of claim 1 or 2, wherein in one subset a standard for the MHC constituent peptide to be screened is used and wherein the method further comprises a step of
(d) comparing the loading efficiency of any subset to be screened to the loading efficiency of the subset including the standard.

4. The method of any preceding claim, wherein loading efficiency is determined by a conformational binding assay using at least one monoclonal antibody not capable of binding to the non-loaded MHC molecule, but capable of binding to the loaded MHC molecule to form an MHC-antibody complex.

5. The method of claim 4, wherein the monoclonal antibody is immobilized on a solid support.

6. The method of any preceding claim which is a cell-free assay.

7. The method of any preceding claim for screening the binding efficiencies of putative MHC binding peptides to MHC molecules present in a crude, unpurified mixture.

8. The method of claim 7, wherein the MHC molecules are oligomers and wherein each oligomer comprises at least two chimeric proteins comprising a first section derived from an MHC constituent peptide or functional part thereof and a second section comprising an oligomer forming coiled-coil domain, wherein formation of the oligomeric MHC molecule occurs by oligomerisation at the oligomerising domain of the chimeric proteins and wherein at least two of the first section of all chimeric proteins comprised in said oligomer are derived from the same MHC peptide chain.

9. The method of claim 8 wherein the oligomerising domain comprised in at least one of the chimeric proteins is derived from the pentamerisation domain of cartilage oligomeric matrix protein (COMP).

10. The method of any preceding claim wherein the MHC molecules contain a recognition sequence for a biotinylating enzyme fused to at least one of their constituent peptides.

11. The method of any of claims 8 or 10, wherein the MHC molecules are tetramers.

12. The method of any of claims 8 to 10, wherein the MHC molecules are pentamers.

13. The method of any preceding claim further comprising the step of providing a set of screened MHC molecules.

## Patentansprüche

1. Verfahren zum Screening nach Bindungseigenschaften von Bestandteilen der Peptide von MHC-Molekülen, wobei das Verfahren die Schritte umfasst:
(a) Bereitstellen von MHC-Molekülen oder Bestandteilen der Peptide davon als einen Satz von MHC-Molekülen, in Lösung, wobei der Satz eine Vielzahl von Teilsätzen von MHC-Molekülen einschließt,
wobei die MHC-Moleküle von jeden Teilsatz sich von den MHC-Molekülen des mindestens einen anderen Teilsatzes in mindestens einem Bestandteil eines Peptids unterscheiden, der ausgewählt ist aus der Gruppe bestehend aus einem mutmaßlichen MHC-Bindungspeptid, einer MHC-alpha-Kette und einer MHC-beta-Kette, und
Beladen der MHC-Moleküle mit einem MHC-Bindungspeptid durch
(i) Rückfalten der MHC-alpha- und beta-Peptidketten in Gegenwart der MHC-Bindungspeptide, oder
(ii) durch Austausch der Peptide oder Beladen mit einem nichtmarkierten MHC-Bindungspeptid in der Abwesenheit eines markierten MHC-Bindungspeptids,
(b) Entnehmen mindestens einer Probe von jeden Teilsatz, und
(c) Bestimmen der Beladungseffizienz der Probe von Schritt (b), wobei die Beladungseffizienz durch Messen des Verhältnisses der beladenen MHC-Moleküle zu den nicht-beladenen MHC-Molekülen bestimmt wird,
wobei die MHC-Moleküle MHC-Oligomere sind, und wobei die Oligomerisierung vor Schritt b) erfolgt, zum Beispiel nach Schritt a) oder parallel zu dem Beladen durch Rückfaltung in Schritt a).

2. Verfahren nach Anspruch 1 zum Screening mutmaßlicher MHC-Bindungspeptide, wobei die MHC-Moleküle jedes Teilsatzes sich bezüglich des mutmaßlichen MHC-Bindungspeptids unterscheiden, das in die MHC-Bindungsfurche zu beladen sind, wobei das Peptid bevorzugt im wesentlichen homogen für jeden Teilsatz ist.

3. Verfahren nach Anspruch 1 oder 2, wobei in einem Teilsatz ein Standard für den zu screenenden MHC-Peptidbestandteil verwendet wird und wobei das Verfahren zudem einen Schritt umfasst:
(d) Vergleichen der Beladungseffizienz von jedem zu screenenden Teilsatz zu der Beladungseffizienz des Teilsatzes, die den Standard beinhaltet.

4. Verfahren nach einem vorhergehenden Anspruch, wobei die Beladungseffizienz durch einen Konformations-Bindungsassay bestimmt wird, der mindestens einen monoclonalen Antikörper verwendet, der nicht fähig ist, an das nicht-beladene MHC-Molekül zu binden, der aber fähig ist, an das beladene MHC-Molekül zu binden, um einen MHC-Antikörper-Komplex zu bilden.

5. Verfahren nach Anspruch 4, wobei der monoclonale Antikörper an einen festen Träger immobilisiert ist.

6. Verfahren nach einem vorhergehenden Anspruch, das ein zellfreier Assay ist.

7. Verfahren nach einem vorhergehenden Anspruch zum Screening der Bindungseffizienzen von mutmaßlichen MHC-Bindungspeptiden an MHC-Moleküle, die in einer kruden, nicht-aufgereinigten Mischung vorliegen.

8. Verfahren nach Anspruch 7, wobei die MHC-Moleküle Oligomere sind und wobei jedes Oligomer mindestens zwei chimäre Proteine umfasst, die einen ersten Abschnitt umfassen, der von einem MHC-Peptidbestandteil oder einem funktionellen Teil davon stammt, und einen zweiten Abschnitt, der eine Oligomer-bildende Coiled-Coil-Domäne umfasst, wobei die Bildung der oligomeren MHC-Moleküle durch Oligomerisierung an der Oligomerisierungsdomäne der chimären Proteine erfolgt, und wobei mindestens zwei des ersten Abschnitts von allen chimären Proteine, die in dem Oligomer umfasst sind, von derselben MHC-Peptidkette strammen.

9. Verfahren nach Anspruch 8, wobei die Oligomerisierungsdomäne, die in mindestens einem der chimären Proteine umfasst ist, von einer Pentamerisierungsdomäne des oligomeren Knorpel-Matrixproteins (cartilage oligomeric matrix protein; COMP) stammt.

10. Verfahren nach einem vorhergehenden Anspruch, wobei die MHC-Moleküle eine Erkennungssequenz für ein biotinylierendes Enzym enthalten, die an mindestens einen von deren Peptidbestandteilen fusioniert ist.

11. Verfahren nach einem der Ansprüche 8 oder 10, wobei die MHC-Moleküle Tetramere sind.

12. Verfahren nach einem der Ansprüche 8 bis 10, wobei die MHC-Moleküle Pentamere sind.

13. Verfahren nach einem vorhergehenden Anspruch, das zudem den Schritt des Bereitstellens eines Satzes von gescreenten Molekülen umfasst.

## Revendications

1. Méthode de criblage des propriétés de liaison des peptides constitutifs de molécules du CMH, ladite méthode comprenant les étapes suivantes :
(a) la fourniture en solution de molécules du CMH ou de leurs peptides constitutifs pour un ensemble de molécules du CMH, lequel ensemble inclut une pluralité de sous-ensembles de molécules du CMH,
dans laquelle les molécules du CMH de chaque sous-ensemble diffèrent des molécules du CMH d'au moins un autre sous-ensemble par au moins un peptide constitutif choisi dans le groupe constitué d'un peptide de liaison du CMH putatif, une chaîne alpha du CMH et une chaîne bêta du CMH, et
le chargement desdites molécules du CMH avec un peptide de liaison du CMH par
(i) repliement des peptides de la chaîne alpha et de la chaîne bêta du CMH en présence dudit peptide de liaison du CMH ou
(ii) par échange de peptide ou chargement avec un peptide de liaison du CMH non marqué en l'absence de tout peptide de liaison du CMH marqué,
(b) le prélèvement d'au moins un échantillon à partir de chaque sous-ensemble, et
(c) la détermination de l'efficacité de chargement pour l'échantillon de l'étape (b), dans laquelle ladite efficacité de chargement est déterminée en mesurant la proportion des molécules du CMH chargées par rapport aux molécules du CMH non chargées,
dans laquelle lesdites molécules du CMH sont des oligomères du CMH et dans laquelle l'oligomérisation se déroule avant l'étape b) comme après l'étape a) ou en parallèle au chargement par repliement dans l'étape a).

2. Méthode selon la revendication 1 pour le criblage de peptides de liaison du CMH putatifs, dans laquelle les molécules du CMH de chaque sous-ensemble diffèrent en ce qui concerne les peptides de liaison du CMH putatifs à charger dans le sillon de liaison du CMH, ledit peptide étant de préférence sensiblement homogène pour chaque sous-ensemble.

3. Méthode selon la revendication 1 ou 2, dans laquelle dans un sous-ensemble, un étalon pour le peptide constitutif du CMH à cribler est utilisé et dans laquelle la méthode comprend en outre une étape de
(d) comparaison de l'efficacité de chargement de tout sous-ensemble à cribler par rapport l'efficacité de chargement du sous-ensemble comprenant l'étalon.

4. Méthode selon l'une quelconque des revendications précédentes, dans laquelle l'efficacité de chargement est déterminée par une analyse conformationnelle de la liaison en utilisant au moins un anticorps monoclonal incapable de se lier à la molécule du CMH non chargée, mais capable de se lier à la molécule du CMH chargée pour former un complexe CMH-anticorps.

5. Méthode selon la revendication 4, dans laquelle l'anticorps monoclonal est immobilisé sur un support solide.

6. Méthode selon l'une quelconque des revendications précédentes qui est une technique acellulaire.

7. Méthode selon l'une quelconque des revendications précédentes pour le criblage des efficacités de liaison de peptides de liaison du CMH putatifs aux molécules du CMH présentes dans un mélange brut non purifié.

8. Méthode selon la revendication 7, dans laquelle les molécules du CMH sont des oligomères et dans laquelle chaque oligomère comprend au moins deux protéines chimériques comprenant une première section dérivée d'un peptide constitutif du CMH ou d'une partie fonctionnelle de celui-ci et une seconde section comprenant un oligomère formant un domaine surenroulé, dans laquelle la formation de la molécule du CMH oligomère a lieu par oligomérisation au niveau du domaine d'oligomérisation des protéines chimériques et dans laquelle au moins deux de la première section de toutes les protéines chimériques comprises dans ledit oligomère sont dérivées de la même chaîne peptidique du CMH.

9. Méthode selon la revendication 8 dans laquelle le domaine d'oligomérisation compris dans au moins l'une des protéines chimériques est dérivé du domaine de pentamérisation de la protéine matricielle oligomère du cartilage (COMP).

10. Méthode selon l'une quelconque des revendications précédentes dans laquelle les molécules du CMH contiennent une séquence de reconnaissance pour une enzyme de biotinylation fusionnée à au moins l'un de leurs peptides constitutifs.

11. Méthode selon l'une quelconque des revendications 8 à 10, dans laquelle les molécules du CMH sont des tétramères.

12. Méthode selon l'une quelconque des revendications 8 à 10, dans laquelle les molécules du CMH sont des pentamères.

13. Méthode selon l'une quelconque des revendications précédentes comprenant en outre l'étape de fourniture d'un ensemble de molécules du CMH criblées.
